# EUROPEAN PATENT APPLICATION

(11) **EP 3 081 638 A1**
(43) Date of publication of application: **19.10.2016**
(21) Application number: 16165439.7
(22) Date of filing: 14.04.2016
(51) Int. Cl.: C12N 5/071

(54) **METHOD FOR PRODUCING PSEUDO-ISLETS**

(30) Priority: 16.04.2015 JP 2015084360
(71) Applicant: Kyoto University, Kyoto-shi, Kyoto 606-8501 (JP); ARKRAY, Inc., Minami-ku Kyoto-shi, Kyoto 601-8045 (JP)
(72) Inventor: IWATA, Hiroo, Kyoto-shi, Kyoto 606-8501 (JP); KONAGAYA, Shuhei, Kyoto-shi, Kyoto 606-8501 (JP); HIRANO, Kunio, Kyoto-shi, Kyoto 602-0008 (JP)
(74) Representative: Golding, Louise Ann

(57) **Abstract**

Provided is a method for producing a pseudoislet, which method comprises: seeding 1,500 to 5,000 pluripotent stem cells in a cell culture well to prepare an aggregate of said pluripotent stem cells, said aggregate having a predetermined size; and culturing said aggregate in said cell culture well in a nonadherent state, to allow differentiation into cells including pancreatic islet progenitor cells and/or pancreatic islet cells.

## Description

### TECHNICAL FIELD

The present invention relates to a method for producing a pseudoislet, which method allows efficient induction of differentiation of pluripotent stem cells, preferably human pluripotent stem cells into pancreatic islet cells.

### BACKGROUND ART

As a therapeutic method for diabetes, especially type I diabetes, pancreas transplantation or pancreatic islet transplantation is effective. However, in such transplantation, there are problems such as a limited number of organs donated, and the necessity of administration of an immunosuppressive agent for prevention of immune rejection. On the other hand, studies on induction of differentiation of pluripotent stem cells such as induced pluripotent stem cell (iPS cells) into pancreatic islet cells have been widely carried out using cells derived from mouse or human.

US 8859286 B shows a method for induction of differentiation into endodermal cells, especially pancreatic islet cells, using stimulating factors such as TGF-β. However, although this document shows stimulating factors necessary for induction of the cell fate into endodermal lineages, especially pancreatic islet cells, whether the induced cell population is kept to have a flat structure or formed into a three-dimensional aggregate is not shown, and no method for controlling the size of the cell population in this process is shown.

JP 2011-115161 A shows a method for culturing embryonic stem cells (ES cells) or iPS cells on a matrix composed of positively-charged, nano-sized fibers or particles, to induce pancreatic islet cells from pluripotent stem cells without using feeder cells. However, there is neither description nor suggestion on formation of aggregates.

DIABETES, VOL. 61, AUGUST 2012, 2016-2029 shows a method in which human pluripotent stem cells are induced to differentiate into pancreatic islet cells, and the differentiated pancreatic islet cells are allowed to form cell aggregates, to prepare pseudoislets. The document also shows that transplantation of the pseudoislets ameliorated the diabetic condition of diabetic model mice. In this document, the differentiation induction was performed by adherent culture of the cells on the bottom surface of a culture dish, and, during the process, cell populations were detached from the bottom surface of the culture dish, and subjected to nonadherent culture (suspension culture) to allow formation of the aggregates. However, since the formation of the aggregates depends of random adhesion of cell populations to each other during the suspension culture, the size of each aggregate cannot be controlled.

JP 5039715 B shows a method and a device in which seeded cells are allowed to form aggregates in recesses constituted by a non-cell-adhesive hydrogel substrate. However, this document does not show a method of their application to differentiation induction from pluripotent stem cells. There are also known methods for inducing differentiation into islet cells using cells in a state of aggregates formed by shake culture, such as the methods described in PLoS ONE., VOL. 7, May 2012, e37004, Thomas C. Schulz et al.; and Cell. VOL. 159, Oct 2014,428-439, Felicia W Pagliuca et al.

JP 2007-135593 A discloses a method for forming cell aggregates in a culture dish the surface of which is modified such that cell adhesion is inhibited, and mentions the cell seeding density and the serum concentration in the medium as factors which largely contribute to the size of each aggregate. The document describes a method in which the area where cell adhesion may occur on the bottom surface of the culture dish is restricted to limit the size of the resulting cell aggregate. However, this document does not show a method of its application to differentiation induction.

### SUMMARY OF THE INVENTION

As described above, methods for inducing pancreatic islet cells from pluripotent stem cells have been conventionally attempted. However, those methods were insufficient for obtaining practical pseudoislets from the viewpoint of efficiency and quality. Moreover, the conventional methods needed laborious operations.

In view of this, an object of the present invention is to provide a method for efficiently inducing differentiation of pluripotent stem cells into pancreatic islet progenitor cells and/or pancreatic islet cells by simple operations, to produce practical pseudoislets which are suitable for transplantation therapy and the like.

In the present description, "pseudoislet" means a tissue prepared from stem cells *in vitro,* unlike a pancreatic islet isolated from the pancreas of a living body. A pseudoislet contains at least insulin-secreting cells, and has, for example, a cell cluster structure. Examples of the pseudoislet include those prepared from stem cells such as embryonic stem cells (ES cells) or induced pluripotent stem cells (iPS cells). Examples of the "pseudoislet" also include those prepared from other pluripotent stem cell such as stem cells contained in a living body.

In order to solve the problems described above, the present invention provides a method for producing a pseudoislet, the method comprising the steps of: seeding 1,500 to 5,000 pluripotent stem cells in a cell culture well (having a diameter of 200 µm to 800 µm and a depth of 400 µm to 1000 µm, for example) to prepare an aggregate of the pluripotent stem cells, the aggregate having a predetermined size; and culturing the resulting aggregate in the cell culture well in a nonadherent state while appropriately changing the medium composition with time, to allow differentiation into a cell cluster containing a pancreatic islet progenitor cell and/or pancreatic islet cell. The present invention can be realized by, for example, using a well having a hemispheric bottom portion as the above-described well. By this, the differentiation step can be carried out using a plurality of wells containing aggregates having almost uniform size. In the present invention, for example, a microwell plate made of a hydrogel may be preferably used.

By the culturing method of the present invention, differentiation of pluripotent stem cells into pancreatic islet progenitor cells and/or pancreatic islet cells can be induced with high efficiency by an operation which is simpler than those in conventional methods. More specifically, the number of seeded cells is controlled to prepare cell aggregates having uniform size, and differentiation induction is carried out in a state where a single cell aggregate is maintained in each well (recess) (that is, in a state where cell aggregates are not in contact with each other). By this, the sizes of the cell aggregates can be controlled to fall within a certain range, and uniform differentiation efficiency can be realized. That is, in the method of the present invention, a high cell survival rate can be realized since oxygen and nutrients easily infiltrate into the center of each aggregate. Moreover, since a single aggregate is maintained in each well to prevent contacting of cell aggregates with each other, and differentiation induction is carried out with cell aggregates in this state, the following advantages can be obtained: 1) detachment of the cells in an undesirable period during the differentiation induction process is not necessary; 2) since there is no need to collect the cells after the certain period of differentiation culture and to form their aggregates, the operation can be simplified; 3) since the only operation necessary before the final stage of the differentiation induction is replacement of the medium, automation is possible; the differentiation induction efficiency can be improved compared to conventional methods using adherent culture; and, 4) since the developmental process in the present method, compared to the processes in conventional techniques, is thought to be more similar to that in a living body, which is accompanied by formation of a three-dimensional structure, it is likely that the present method can induce islet cells having properties more similar to those of adult pancreatic islets.

### BRIEF DESCRIPTION OF THE DRAWINGS

Fig. 1 is an embodiment of a culture device which can be used in the method of the present invention.
Fig. 2 is a diagram showing the conditions for the induction of differentiation of iPS cells into pancreatic islet cells in Examples.
Fig. 3 (A) shows photographs showing morphologies of aggregates during the differentiation induction process, and morphologies of cell aggregates recovered from the agarose well plate. Fig. 3 (B) shows a graph showing a distribution of diameters of cell aggregates during the differentiation induction process.
Fig 4 shows photographs showing the results of immunostaining of aggregates on Day 3 (a) of the differentiation induction and on Day 20 (b) of the differentiation induction.
Fig. 5 shows a diagram showing the results of investigation of insulin secretion using the aggregates obtained on Day 34 of the differentiation induction.
Fig. 6 shows diagrams (photographs) showing the differences in the survival rate depending on the number of seeded cells in the preparation of cell aggregates.
Fig. 7 shows diagrams showing the results (flow cytometry, FACS) of comparison of the efficiency of differentiation into embryonic endodermal cells in the early stage of the induction by aggregate culture or adherent culture.
Fig. 8 shows diagrams showing the results (FACS) of comparison of the efficiency of differentiation into islet cells in the late stage of the induction by aggregate culture or adherent culture.
Fig. 9 shows diagrams showing the results (RT-PCR) of comparison of the efficiency of differentiation into islet cells in the late stage of the induction by aggregate culture or adherent culture. In each panel, the result of the adherent culture is shown in the left side, and the result of the aggregate culture is shown in the right side.

### EMBODIMENTS FOR CARRYING OUT THE INVENTION

### <Method of Producing Pseudoislet>

### (Pluripotent Stem Cells)

In the present invention, the pluripotent stem cells are stem cells having pluripotency that allows differentiation into many kinds of cells present in a living body, which stem cells also have the growth ability by self-renewal. The pluripotent stem cells at least include cells which can be differentiated into the islet progenitor cells. Examples of the pluripotent stem cells include, but are not limited to, embryonic stem (ES) cells, embryonic stem cells derived from a cloned embryo obtained by nuclear transfer (ntES cells), multipotent germline stem cells ("mGS cells"), embryonic germ cells ("EG cells"), and induced pluripotent stem (iPS) cells. The pluripotent stem cells are preferably iPS cells since these cells can be obtained without destroying embryos, eggs, or the like during the production process.

Methods for producing iPS cells are known in the art. These cells can be produced by introducing reprogramming factors into somatic cells. Examples of the reprogramming factors herein include genes such as *Oct3*/*4, Sox2, Sox1, Sox3, Sox15, Sox17, Klf4, Klf2, c-Myc, N-Myc, L-Myc, Nanog, Lin28, Fbx15, ERas, ECAT15-2, Tel1, beta-catenin, Lin28b, Sall1, Sall4, Esrrb, Nr5a2, Tbx3,* and *Glis1,* and gene products thereof. These reprogramming factors may be used individually, or two or more of these may be used in combination. Examples of the combination of the reprogramming factors include those described in WO 2007/069666; WO 2008/118820; WO 2009/007852; WO 2009/032194; WO 2009/058413; WO 2009/057831; WO 2009/075119; WO 2009/079007; WO 2009/091659; WO 2009/101084; WO 2009/101407; WO 2009/102983; WO 2009/114949; WO 2009/117439; WO 2009/126250; WO 2009/126251; WO 2009/126655; WO 2009/157593; WO 2010/009015; WO 2010/033906; WO 2010/033920; WO 2010/042800; WO 2010/050626; WO 2010/056831; WO 2010/068955; WO 2010/098419; WO 2010/102267; WO 2010/111409; WO 2010/111422; WO 2010/115050; WO 2010/124290; WO 2010/147395; WO 2010/147612; Huangfu D, et al. (2008), Nat. Biotechnol., 26: 795-797; Shi Y, et al. (2008), Cell Stem Cell, 2: 525-528; Eminli S, et al. (2008), Stem Cells. 26:2467-2474; Huangfu D, et al. (2008), Nat. Biotechnol. 26:1269-1275; Shi Y, et al. (2008), Cell Stem Cell, 3, 568-574; Zhao Y, et al. (2008), Cell Stem Cell, 3:475-479; Marson A, (2008), Cell Stem Cell, 3, 132-135; Feng B, et al. (2009), Nat. Cell Biol. 11:197-203; R.L. Judson et al., (2009), Nat. Biotechnol., 27:459-461; Lyssiotis CA, et al. (2009), Proc Natl Acad Sci USA. 106:8912-8917; Kim JB, et al. (2009), Nature. 461:649-643; Ichida JK, et al. (2009), Cell Stem Cell. 5:491-503; Heng JC, et al. (2010), Cell Stem Cell. 6:167-74; Han J, et al. (2010), Nature. 463:1096-100; Mali P, et al. (2010), Stem Cells. 28:713-720; and Maekawa M, et al. (2011), Nature. 474:225-9.

Examples of the somatic cells include, but are not limited to, any of fetal somatic cells, neonatal somatic cells, and healthy or diseased adult somatic cells, as well as any of primary cultured cells, subcultured cells, and established cell lines. Specific examples of the somatic cells include (1) tissue stem cells (somatic stem cells) such as neural stem cells, hematopoietic stem cells, mesenchymal stem cells, and dental pulp stem cells; (2) tissue progenitor cells; and (3) differentiated cells such as blood cells (peripheral blood cells, cord blood cells, and the like), lymphocytes, epithelial cells, endothelial cells, muscle cells, fibroblasts (skin cells and the like), hair cells, hepatic cells, gastric mucosal cells, enterocytes, spleen cells, pancreatic cells (pancreatic exocrine cells and the like), brain cells, lung cells, kidney cells, and adipocytes. It is expected that, in cases where the iPS cells to be used for the preparation of pseudoislets are prepared from somatic cells derived from the patient himself to which the pseudoislets are to be applied, preparation of pseudoislets for transplantation which do not cause immune rejection may be possible.

### (Culture Device)

The culture device to be used in the method of the present invention has culture wells each having a size that allows formation of a cell aggregate by seeding 1,500 to 5,000 cells/well, preferably 2,000 to 3,000 cells/well. The method of the present invention can be realized using a culture well having a capacity of, for example, 0.001 µl/well to 10 µl/well, 0.001 to 1 µl/well, 0.005 µl/well to 0.5 µl/well, 0.01 µl/well to 0.5 µl/well, or 0.01 µl/well to 0.1 µl/well. The method of the present invention can be realized using a culture well having a shape that easily allows precipitation of cells into the bottom portion and formation of their aggregate, for example, a hemispheric culture well whose bottom portion expands toward the bottom, or a cylindrical culture well having a spherical bottom portion. The diameter of such a culture well is, for example, 200 µm to 800 µm, or 400 to 800 µm. The depth of such a culture well is, for example, 400 µm to 1000 µm, or 400 to 800 µm. A large number of pseudoislets can be obtained using a multiwell culture device having a plurality of wells each of which has a shape described above.

Since nonadherent culture is carried out, the culture device may have a culture surface subjected to cell-non-adhesive treatment. However, the culture device is preferably made of a material which allows cell culture in a nonadherent state. Such a material is preferably a non-cytotoxic hydrophilic material having a three-dimensional structure. The material is more preferably a transparent material from the viewpoint of enabling easy observation of the culture state. More specifically, the material is preferably a hydrogel.

Examples of the material used for preparing the hydrogel include synthetic polymers that can form hydrogels, such as products prepared by chemical cross-linking or radiation cross-linking of synthetic polymers including polyvinyl alcohol, polyvinyl pyrrolidone, polyethylene glycol, poly-2-hydroxyethyl methacrylate, poly-2-hydroxyethyl acrylate, polyacrylamide, polyacrylic acid, and polymethacrylic acid; and products prepared by cross-linking of copolymers prepared with monomers constituting the polymers described above. Polysaccharides such as agarose, alginic acid, dextran, and cellulose as natural polymers, and derivatives thereof; and cross-linked products of proteins such as gelatin and albumin, and derivatives thereof; may also be used.

### (Culture Method)

An embodiment of the culture method of the present invention is described below.

First, pluripotent stem cells cultured in an adherent state in advance are detached from the culture dish, and dissociated into single cells. This process can be carried out by, for example, using an enzyme such as trypsin. The pluripotent stem cells dissociated into single cells are suspended in a medium, and seeded in a culture vessel such that the cell concentration becomes 1,500 to 5,000 cells/well. The cells are then left to stand in this state for a certain period, for example, for 12 to 36 hours, to allow formation of aggregates.

The medium to be used for allowing the formation of aggregates may be a medium commonly used for culture of pluripotent stem cells. The medium is preferably supplemented with Rho-associated coiled-coil forming kinase (ROCK) inhibitor. The culture conditions during the formation of the aggregates may be the same as those for ordinary cell culture. The culture temperature is preferably 35 to 39°C, more preferably 37°C. The culture is preferably carried out under ordinary conditions in the presence of about 5 to 20% O₂ and about 5% CO₂. The culture period is not limited as long as the aggregates can be formed during the period. For example, the culture period is 10 to 30 hours.

Subsequently, by changing of the medium composition, the pluripotent stem cells forming aggregates having a predetermined size, for example, a diameter of 100 µm to 500 µm, are stimulated to cause their differentiation into cell clusters containing pancreatic islet progenitor cells and/or pancreatic islet cells.

The medium in this process can be prepared by adding necessary differentiation-inducing factors to a basal medium which is a medium used for culturing animal cells. Examples of the basal medium include IMDM, Medium 199, Eagle's Minimum Essential Medium (EMEM), αMEM, Dulbecco's modified Eagle's Medium (DMEM), Ham's F12 medium, RPMI 1640 medium, Fischer's medium, MCDB 131 medium, E8 medium, mTeSRI medium, and mixtures of two or more of these media. The medium may contain serum, or may be serum-free. If necessary, the medium may contain one or more of serum replacements such as albumin, transferrin, Knockout Serum Replacement (KSR) (serum replacement for fetal bovine serum (FBS) in ES cell culture), fatty acids, insulin, collagen precursor, trace elements, 2-mercaptoethanol, 3'-thiolglycerol, and ITS-supplement, and may also contain one or more of substances such as B27-supplement, N2-supplement, lipids, amino acids, L-glutamine, Glutamax (Invitrogen), non-essential amino acids, vitamins, growth factors, cytokines, antibiotics, antioxidants, pyruvic acid, buffers, and inorganic salts.

In the process of allowing differentiation of pluripotent stem cells into pancreatic islet progenitor cells and/or pancreatic islet cells, the composition of the medium may be changed with time such that the process of pancreatic development in a living body is mimicked.

Examples of such a method include the methods described in the following documents, and appropriately modified methods thereof.
[Document 1] Rezania A, Bruin JE, Riedel MJ, Mojibian M, Asadi A, Xu J, Gauvin R, Narayan K, Karanu F, O'Neil JJ, Ao Z, Warnock GL, Kieffer TJ. Maturation of human embryonic stem cell-derived pancreatic progenitors into functional islets capable of treating pre-existing diabetes in mice. Diabetes 2012; 61:2016-2029.
[Document 2] Rezania A, Bruin JE, Arora P, Rubin A, Batushansky I, Asadi A, O'Dwyer S, Quiskamp N, Mojibian M, Albrecht T, Yang YH, Johnson JD, Kieffer TJ. Reversal of diabetes with insulin-producing cells derived in vitro from human pluripotent stem cells. Nat Biotechnol 2014; 32:1121-1133.
[Document 3] Hrvatin S, O'Donnell CW, Deng F, Millman JR, Pagliuca FW, DiIorio P, Rezania A, Gifford DK, Melton DA. Differentiated human stem cells resemble fetal, not adult, β cells. Proc Natl Acad Sci U S A. 111,3038-3043 (2014)
[Document 4] Pagliuca FW, Millman JR, Gurtler M, Segel M, Van Dervort A, Ryu JH, Peterson QP, Greiner D, Melton DA. Generation of functional human pancreatic β cells in vitro. Cell. 2014; 159:428-439.

For example, Activin A and/or Wnt3a is/are preferably added in the early stage, and it is also preferred to add retinoic acid, hedgehog signaling inhibitor (e.g., SANT-1 or Cyclopamine-KAAD), and/or fibroblast growth factor thereafter.

In the process of differentiation, in order to obtain functional pancreatic islets by mimicking the process of pancreatic development in a living body, one or more of substances that maintain the undifferentiated state while promoting the growth, substances that suppress the growth while promoting the differentiation, proteins expressed in the pancreas in a living body, and substances that promote insulin secretion, may be added at an appropriate timing(s). Examples of such substances include GSK-3β (Glycogen Synthase Kinase 3β) inhibitors (e.g., CHIR99021), ALK inhibitors (e.g., SB431542), Notch signaling inhibitors (e.g., DAPT), TGFβ inhibitors (e.g., LDN193189), AMPK and BMP signaling inhibitors (e.g., Dorsomorphin), PKC activators (e.g., Pdbu), insulin-like growth factor-1, epidermal growth factors, hepatocyte growth factors, glucagon-like peptide-1, and commercially available supplements.

The culture may be continued for a period of time which allows the aggregates to contain sufficient insulin-producing cells. Examples of the culture period include 20 to 40 days after the beginning of the differentiation stimulation. Preferred examples of the culture period include 25 to 35 days after the beginning of the differentiation stimulation.

The culture temperature is preferably 35 to 39°C, more preferably 37°C. The culture is preferably carried out under ordinary conditions in the presence of about 5 to 20% O₂ and about 5% CO₂.

The medium is preferably periodically replaced, more preferably replaced every day. In cases where the culture is carried out in a microplate having microwells, the microplate may be placed in a petri dish or the like, and the medium may then be placed in the petri dish such that the wells in the microplate are impregnated with the medium. This allows easy replacement of the medium. The medium can be supplied, for example, by changing a medium to a fresh medium using a pipette etc. every day or every few days, by supplying a medium at a constant rate through a channel connected to a culture substrate using a liquid pump, or by activating a liquid pump for a certain period of time to supply a medium through a channel connected to a culture substrate and then stop the pump to carry out static culture.

The differentiation step is preferably carried out in a plurality of wells each containing an aggregate having almost uniform size. The size of the aggregate is, for example, 100 µm to 500 µm, preferably 100 µm to 400 µm, more preferably 200 µm to 400 µm, further more preferably 200 to 350 µm, still more preferably 250 µm to 350 µm in diameter at the beginning of the differentiation induction. After the beginning of the differentiation induction, the aggregate in each well is maintained such that it is preferably 0.5 to 2 times, more preferably 0.8 to 1.5 times the size of the aggregate at the beginning of the differentiation induction.

The "pseudoislet" obtained by the method of the present invention is a cell aggregate which is artificially formed. The "pseudoislet" preferably contain an α-cell, which secretes glucagon, β-cell, which secretes insulin, and δ-cell, which secretes somatostatin, and has a three-dimensional structure similar to that of a pancreatic islet in a living body. The "pseudoislet" preferably has a glucose-responsive insulin-secreting capacity. The presence of α-cells, β cells, and δ cells among islet cells can be confirmed by immunostaining using antibodies against glucagon, insulin, and somatostatin, respectively. β-cells can also be detected by immunostaining using an antibody against C-peptide. C-peptide is a peptide produced when proinsulin, which is a precursor of insulin, is enzymatically degraded into insulin. Alternatively, β-cells may be detected by dithizone staining.

The pseudoislet may also contain a pancreatic polypeptide (PP)-secreting cell and/or islet progenitor cell in addition to an α-cell, which secretes glucagon, β-cell, which secretes insulin, and δ-cell, which secretes somatostatin. In the present description, the "pancreatic islet progenitor cell" means a cell which differentiates into an islet cell in the future. The pancreatic islet progenitor cell may be, for example, a PDX1 (pancreas duodenal homeobox gene 1)-positive, PTF1a (pancreas transcription factor 1a)-positive cell. Positivity of NKX6.1 may also be used as an index. Positivity of PDX1 and positivity of NKX6.1, or positivity of PTF1a and positivity of NKX6.1, may also be used as an index.

Pseudoislets obtained by the method of the present invention can be favorably used for transplantation therapy for patients with diabetes. The method for producing pseudoislets according to the present invention is applicable to induced pluripotent stem cells derived from patients with diabetes (especially type I diabetes). Islet cells obtained by the present method is useful for various studies such as elucidation of the pathogenic mechanism of diabetes and search for new drugs.

### EXAMPLES

The method of producing pseudoislets of the present invention is described below with reference to Examples. However, the embodiments of the present invention are not limited to the following embodiments.

### 1. Providing Agarose Microwell

Agarose microwells were prepared using a 3D Petri Dish manufactured by Microtissues, Inc. by reference to the protocol provided by the manufacturer (www.funakoshi.co.jp/contents/5556).

More specifically, the agarose microwells were prepared by the following procedure.

For small cell aggregates, a mold for 256 wells/plate having a well diameter of 400 µm was used. For large cell aggregates (not less than 3000 cells/well), a mold for 81 wells/plate having a well diameter of 800 µm was used.

First, a warmed agarose solution (2.5% agarose/physiological saline) is poured into the mold.

Subsequently, the mold is allowed to cool to room temperature, and, after gelation of the agarose, the resulting agarose microwells were removed from the mold.

The agarose microwells were transferred to a 12-well plate for cell culture, and a medium (DMEM/F12) was added in the vicinity of the agarose microwells to immerse the agarose microwells therein.

The 12-well plate was then placed in an incubator (37°C, 5% CO₂) to allow equilibration of the agarose plate with the medium in its vicinity.

By this, agarose microwells having 256 wells each of which has a cylindrical portion with a diameter of 400 µm and a depth of 800 µm (or, for large aggregates, 81 wells each of which has a cylindrical portion with a diameter of 800 µm and a depth of 800 µm), and has a hemispheric bottom portion, were obtained.

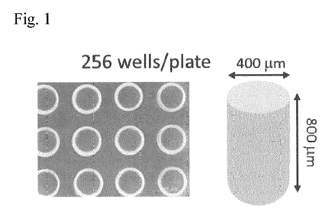

Fig. 1 shows the agarose microwells, and the shape of each microwell.

### 2. Formation of Aggregates, and Differentiation Induction

Using a culture container coated with Geltex (Life Technologies, Inc.), iPS cells (obtained from 253G1 Riken Cell Bank) were cultured for 3 to 4 days in E8 medium (Life Technologies, Inc.).

In a state of 70 to 80% confluence, the cells were detached using TrypLE (Life Technologies, Inc.), and dissociated into single cells. The cells were then suspended in E8 medium supplemented with 10 µM Y-27632 (ROCK inhibitor, Wako Pure Chemical Industries, Ltd.), and seeded at 2500 cells/well, 1250 cells/well, or 5000 cells/well in the agarose microwell plate having 256 wells which was prepared as described above and placed in the well of the 12-well plate.

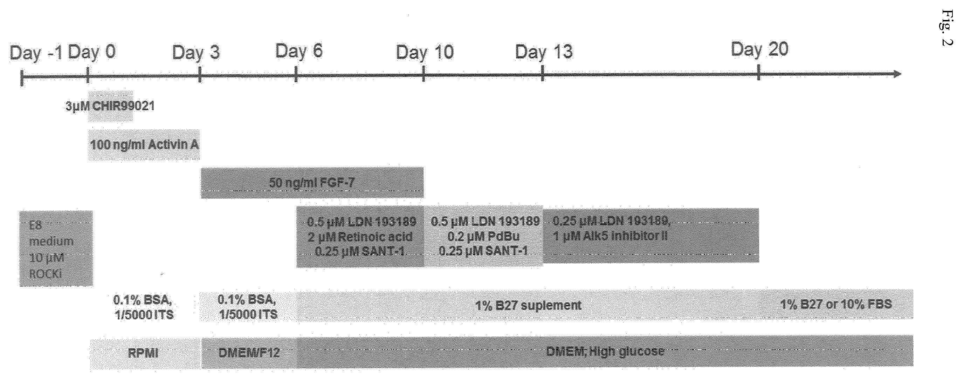

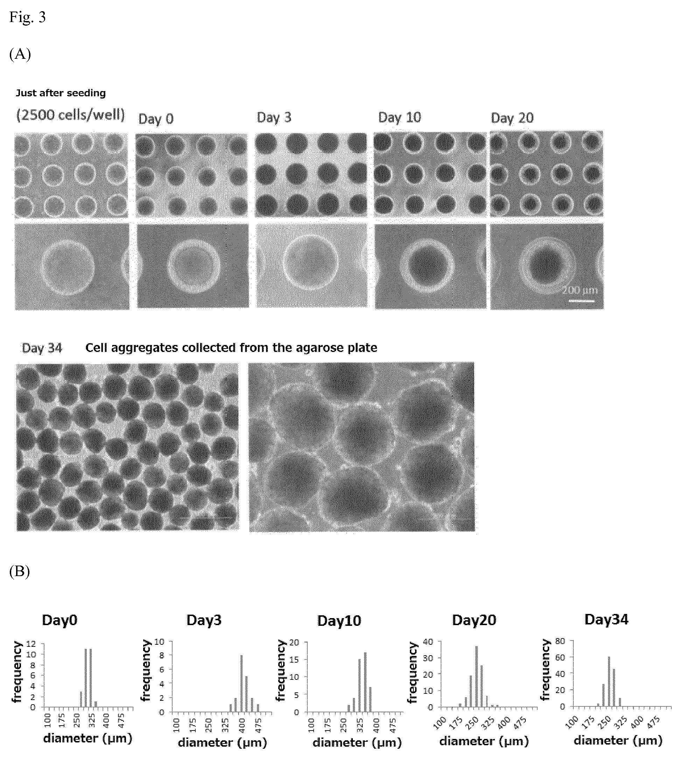

The agarose microwell plate was then left to stand for 10 minutes to allow precipitation of the cells into the bottoms, and a medium (E8 medium + ROCK inhibitor) was then added in the vicinity of the agarose plate to immerse the plate containing the cells in the medium. The cells were cultured at 37°C in 5% CO₂ for 24 hours to allow aggregation of the cells, and then subjected to differentiation induction by the procedure shown in Fig. 2. More specifically, the medium composition was changed with time as described below. Replacement of the medium was carried out every day by sucking. The size of each aggregate immediately before the differentiation induction was about 200 µm to 350µm. The same scale is used for the images shown in the bottom row of Fig. 3, which were taken immediately after the seeding and on Day 0, Day 3, Day 10, and Day 20.

### First Stage (3 Days)

RPMI + 1.2 g/L NaHCO₃, 0.1% delipidated BSA, 1/5000 ITS supplement, 3 µM CHIR99021, 100 ng/mL Activin A (CHIR99021 was added to the medium only on the first day).

### Second Stage (3 Days)

DMEM/F12 + 0.1% delipidated BSA + 1/5000 ITS supplement + 50 ng/mL FGF-7

### Third Stage (4 Days)

DMEM + 1% B27 supplement + 50 ng/mL FGF-7 + 0.25 µM SANT-1 + 0.5 µM LDN193189 + 2 µM retinoic acid

### Fourth Stage (3 Days)

DMEM + 1% B27 supplement + 0.25 µM SANT-1 + 0.5 µM LDN193189 + 0.2 µM PdBu (phorbol 12,13-dibutyrate)

### Fifth Stage (7 Days)

DMEM + 1% B27 supplement + 1 µM Alk5 inhibitor + 0.25 µM LDN193189

### Sixth Stage (14 Days)

DMEM + 1% B27 supplement or DMEM + 10% FBS

Fig. 3(A) shows images of cell aggregates during the differentiation induction process (top row) and morphology of cell aggregates collected from the agarose plate on Day 34 after the differentiation (bottom row). It can be seen that the diameter of each aggregate increased in the early stage of the differentiation, and then gradually decreased to form a more compact aggregate. Fig. 3(B) shows the distribution of diameters of the cell aggregates during the differentiation induction process. The diameters of the cell aggregates were about 250µm to 350µm at the beginning of the differentiation induction (Day 0), about 350µm to 475µm after 3 days from the beginning of the differentiation induction (Day 3), about 250µm to 400µm after 10 days from the beginning of the differentiation induction (Day 10), about 175µm to 350µm after 20 days from the beginning of the differentiation induction (Day 20), and about 200µm to 325µm after 34 days from the beginning of the differentiation induction (Day 34).

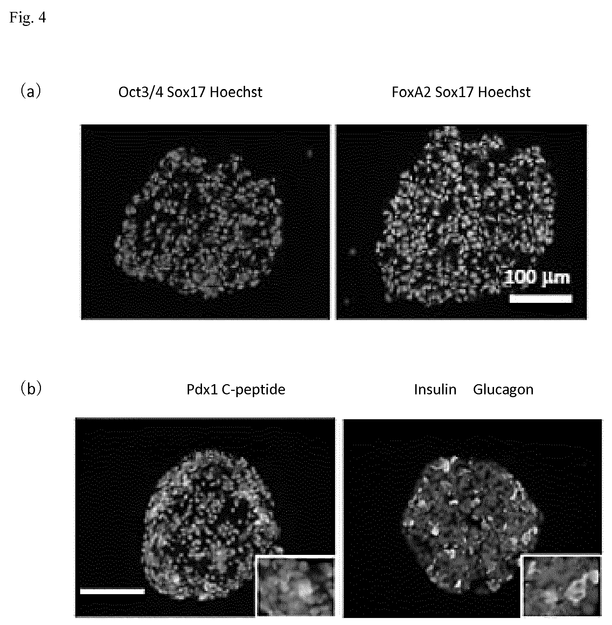

Fig. 4 shows the results of immunostaining of aggregates on Day 3 of the differentiation induction (a) and Day 20 of the differentiation induction (b). It was found that differentiation into embryonic endoderms (SOX17- and FOXA2-positive) occurred with an efficiency of 90% by Day 3 of the culture, and that differentiation into insulin-producing islet cells occurred by Day 20 of the culture.

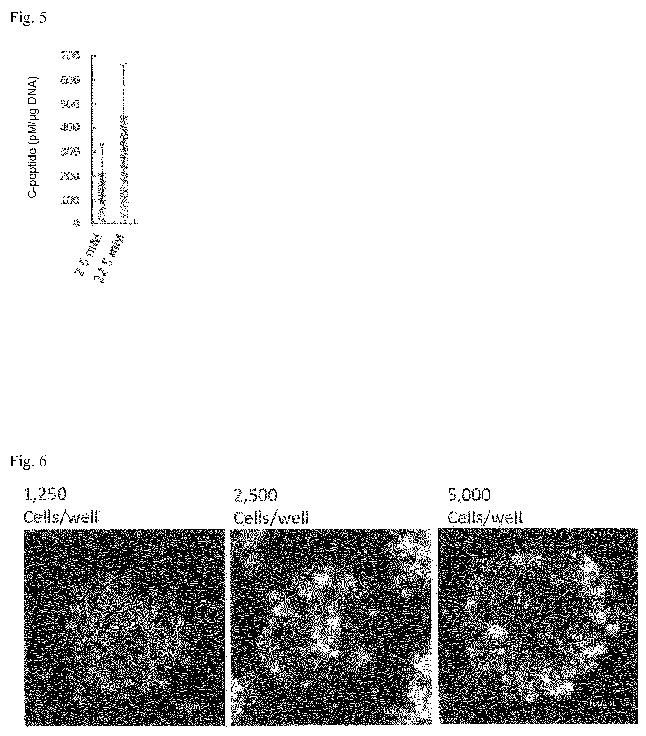

Using aggregates obtained on Day 34 of the differentiation induction, insulin secretion was investigated. As a result, insulin secretion dependent on the glucose concentration could be observed (Fig. 5).

Fig. 6 shows the results of a cell survival assay of islet cell aggregates obtained on Day 18 of the differentiation induction which was carried out with an initial cell concentration of 1250 cells/well, 2500 cells/well, or 5000 cells/well. Living cells were stained with calcein-AM, and dead cells were stained with propidium iodide (PI).

As a result, in the case where the seeding was carried out with an initial cell concentration of 1250 cells/well, most cells in the aggregates died. In the case where the seeding was carried out with an initial cell concentration of 5000 cells/well, a large number of dead cells were found in the center portion of each aggregate. In contrast, in the case where the seeding was carried out with an initial cell concentration of 2500 cells/well, dead cells were hardly found in the center portion of each aggregate.

From these results, it was found that, in cases where the number of cells is small, a large number of cells die during the differentiation induction, while in cases where the aggregate is too large, oxygen and nutrients do not reach the center potion, leading to decreases in the survival rate and the differentiation efficiency in the center portion of the aggregate. It was thus found that the seeding concentration needs to be within a certain range in order to obtain highly active pancreatic islet cells.

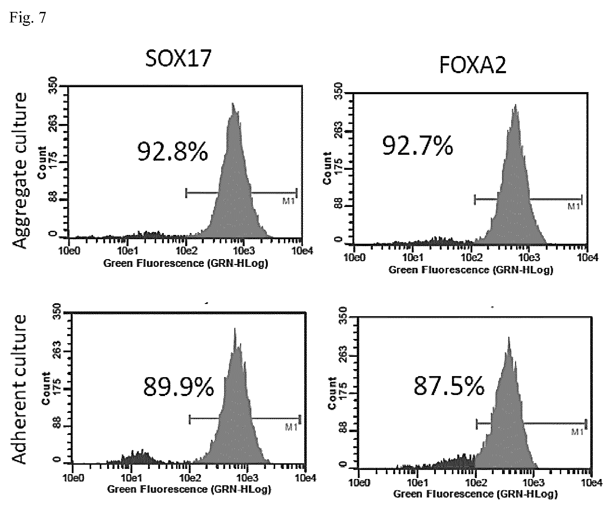

Subsequently, the efficiency of differentiation into embryonic endodermal cells observed in the early stage (Day 3) of the differentiation induction was compared between a case of differentiation by aggregation culture (the method of the present invention) and a case of differentiation by adherent culture (Comparative Example). Fig. 7 shows results of FACS for SOX17 and FOXA2, which are markers for embryonic endodermal cells. From these results, it was found that a better efficiency of differentiation into embryonic endodermal cells can be obtained by differentiation by the aggregation culture.

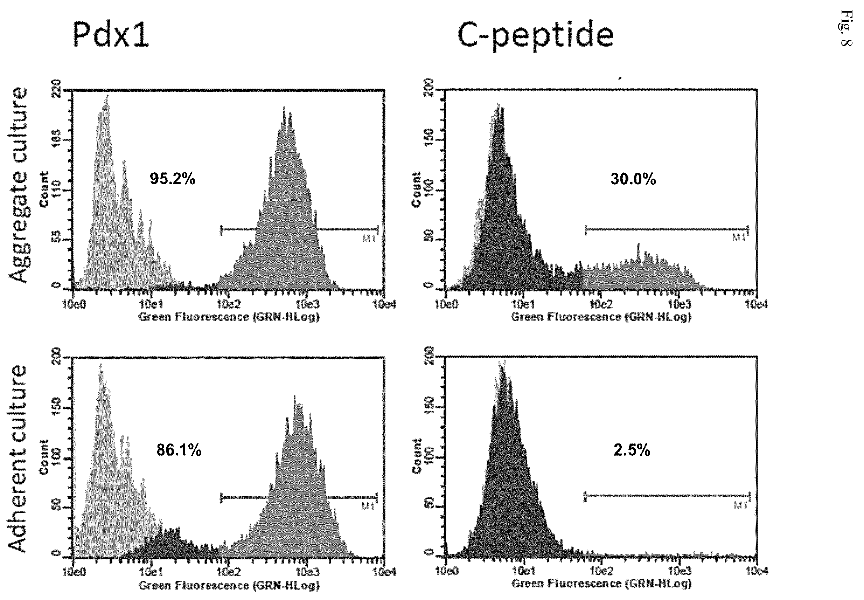

In addition, the efficiency of differentiation into pancreatic islet cells observed in the late stage (Day 20) of the induction was compared between a case of differentiation by aggregation culture (the method of the present invention) and a case of differentiation by adherent culture (Comparative Example). Fig. 8 shows results of FACS for PDX1 and C-peptide, which are markers for islet cells. From these results, it was found that a better efficiency of differentiation into pancreatic islet cells can be obtained by differentiation by the aggregation culture.

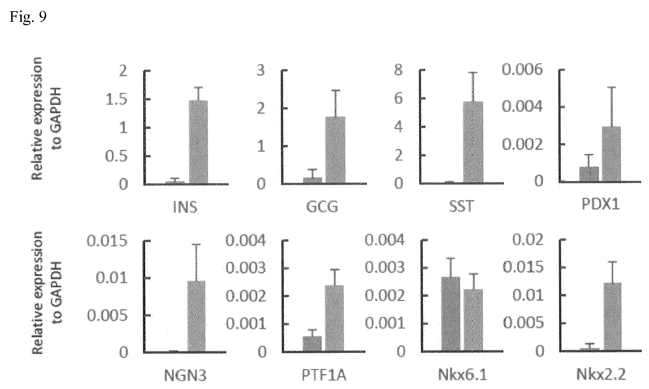

Similarly, expression of marker genes for pancreatic islets was investigated by quantitative RT-PCR for pancreatic islets obtained by the method of the present invention (20 days after the differentiation) and pancreatic islets obtained by differentiation by adherent culture (Comparative Example, 20 days after the differentiation). The results are shown in Fig. 9. As a result, the pancreatic islets obtained by the method of the present invention showed a higher expression level for any of insulin (INS), glucagon (GCG), somatostatin (SST), PDX1, NGN3, PTF1A, Nkx6.1, and Nkx2.2. It was therefore found that a higher efficiency of differentiation into islet cells can be obtained by differentiation by the aggregation culture.

It was also found that expression of exocrine markers also increased in the pancreatic islets obtained by the differentiation by adherent culture (data not shown).

From these results, pancreatic islet cells can be more efficiently induced by the present invention, in which the number of seeded cells is controlled to prepare cell aggregates having uniform size, and differentiation induction is carried out in a state where a single cell aggregate is maintained in each well (that is, in a state where cell aggregates are not in contact with each other), compared to cases where differentiation induction is carried out by conventional adherent culture.

## Claims

1. A method for producing a pseudoislet, comprising:
seeding 1,500 to 5,000 pluripotent stem cells in a cell culture well to prepare an aggregate of said pluripotent stem cells, said aggregate having a diameter of 200 µm to 350 µm; and
culturing said aggregate in said cell culture well in a nonadherent state, to allow differentiation into cells including pancreatic islet progenitor cells and/or pancreatic islet cells.

2. The method according to claim 1, wherein said well has a hemispheric bottom portion.

3. The method according to claim 1 or 2, wherein the capacity of said well is 0.001 µl/well to 10 µl/well.

4. The method according to any one of claims 1 to 3, wherein said well comprises a hydrogel.

5. The method according to any one of claims 1 to 4, wherein said culturing step for differentiation is carried out in a plurality of wells containing the aggregates having almost uniform size.
